Europäisches Patentamt

European Patent Office

Office européen des brevets

(11) Veröffentlichungsnummer: **0 032 165**
**B1**

## (12) EUROPÄISCHE PATENTSCHRIFT

(45) Veröffentlichungstag der Patentschrift: **28.12.83**

(51) Int. Cl.³: **A 61 F 1/03**

(21) Anmeldenummer: **80106674.7**

(22) Anmeldetag: **30.10.80**

(54) Blattartiger Schaft für die Verankerung einer Hüftgelenkprothese.

(30) Priorität: **14.01.80 CH 257/80**

(43) Veröffentlichungstag der Anmeldung:
**22.07.81 Patentblatt 81/29**

(45) Bekanntmachung des Hinweises auf die Patenterteilung:
**28.12.83 Patentblatt 83/52**

(84) Benannte Vertragsstaaten:
**AT DE FR GB IT**

(56) Entgegenhaltungen:
**EP - A - 0 027 160**
**DE - A - 2 746 664**
**DE - B - 2 324 865**
**FR - A - 2 057 418**
**GB - A - 1 409 054**

**Orthopäde 8 (1979), Seiten 73-74**

(73) Patentinhaber: **GEBRÜDER SULZER
AKTIENGESELLSCHAFT
Zürcherstrasse 9
CH-8401 Winterthur (CH)**

(72) Erfinder: **Zweymüller, Karl, Dr.
Döblinger Haupstrasse 22/5
A-1190 Wien (AT)**

(74) Vertreter: **Dipl.-Ing. H. Marsch Dipl.-Ing. K.
Sparing Dipl.-Phys.Dr. W.H. Röhl Patentanwälte
Rethelstrasse 123
D-4000 Düsseldorf (DE)**

Courier Press, Leamington Spa, England.

## Blattartiger Schaft für die Verankerung einer Hüftgelenkprothese

Die Erfindung betrifft einen blattartigen Schaft für die Verankerung einer Hüftgelenkprothese im Femur, wobei der Schaft sich vom distalen freien Ende aus in Richtung seiner Längsmittelachse bis in einen Bereich zwischen etwa 2/3 und 3/4 der Schafthöhe—gemessen entlang der Längsmittelachse—allseitig konisch erweitert und wobei ferner seine mediale Schmalseite aus dem Konus heraus in eine stetig gekrümmte Kurve übergeht, die an einem kragenartigen Ansatz endet, der die Blattseiten vom Prothesenhals trennt.

Unter der "Schafthöhe" wird dabei die Ausdehnung des Schaftes vom distalen Ende bis zur proximalen "Spitze" verstanden, wobei diese Ausdehnung entlang der Längsmittelachse des Schaftes gemessen wird.

Ein Prothesenschaft der vorstehend beschriebenen Art ist aus der unter Art. 54 (3) EPÜ fallenden EP—A—0027160 bekannt; eine sehr ähnliche Konstruktion, bei der der Bogen der medialen Schmalseite des Schaftes, vom distalen Ende her gesehen, bereits früher beginnt, zeigt die DE—A—27 46 664.

Die blattartigen Schäfte werden vor allem für eine zementarme oder zementfreie Verankerung verwendet, um ein Verklemmen im Femurknochen zu erreichen. Damit ist eine gewisse Mindestdicke des Schaftblattes notwendig. Die Implantation des Schaftteiles einer Hüftprothese erfolgt üblicherweise nach Resektion des Hüftkopfes sowie eines anschliessenden Teils des Schenkelhalses. Beim Vollständigen Einsetzen bzw. Einschlagen der konventionellen Prothesenschäfte kommt es durch die erforderliche Mindestdicke des Schaftblattes häufig zu einer sogenannten "Schlussrotation" des Schaftes im Femurknochen, da infolge der mehrfachen Krümmungen des proximalen Femurendes ein gerader oder auch leicht gebogener Gegenstand von der Wand des Oberschenkelknochens abgelenkt wird. Der Schaft sucht sich somit den Weg des geringsten Widerstandes, sofern nicht der Schenkelhals zum Grossteil oder zur Gänze entfernt wird. Selbst wenn man den Verlust an Knochensubstanz in Kauf nehmen würde, ist es bei dem blattartigen Schaft nicht möglich, die Resektionsebene am Schenkelhals zu tief zu legen, um den nötigen Weg zu schaffen. Denn die Blattseiten sollten bei einem blattartigen Schaft parallel zur Querachse des Kniegelenkes verlaufen, um einerseits eine genormte Implantation zu ermöglichen und andererseits eine ideale Stellung des Kopfes der Schaftprothese zur ebenfalls ersetzten Gelenkpfanne zu gewährleisten. Ein zu tiefer Resektionsschnitt führt dazu, dass die üblicherweise annäherndrechteckig geformte Resektionsfläche des Schenkelhalses in ihrem Längsdurchmesser nicht mehr parallel zur Kniegelenkachse steht und somit die Implantation des Schaftes erschwert ist. Damit die Blattseiten der Prothese aber parallel zur Kniegelenksachse zu liegen kommen, muss die Resektion in mittlerer Höhe des Schenkelhalses durchgeführt werden.

Aufgabe der Erfindung ist es, die erwähnte "Schluss-Rotation" beim Einsetzen der Prothese zu vermeiden, ohne die Resektionsebene am Schenkelhals zu tief ansetzen zu müssen. Diese Aufgabe wird erfindungsgemäss dadurch gelöst, dass in Richtung quer zu den Blattbreitseiten das Oberteil des Schaftblattes bis zum kragenartigen Ansatz relativ zum proximalen Ende des Konus verjüngt ist.

Mit der erfindungsgemässen Massnahme wird der Platzbedarf des Oberteils des Schaftblattes im Femurknochen entscheidend verringert, ohne dass die mechanischen Eigenschaften der Prothese, insbesondere ihre Festigkeit, im Konusbereich des Schaftblattes und die Klemmwirkung und Abstützung des Konus im Femurknochen beeinträchtigt werden.

Zusätzlich wird die Gefahr einer Schaftsprengung, wie sie bei raumfordernden Implantaten mit im oberen Anteil eher quadratischen Schaftquerschnitten auftreten können, deutlich reduziert. Wegen der zwei verschiedenen Krümmungen des oberen Endes des Oberschenkelknochens (von der Seite her gesehen S-förmig), nämlich der Antetorsion des Schenkelhalses (Drehung nach vorne), sowie der Antekurvation des Schaftes (Biegung mit der Konvexität nach vorne), ist der Einbau der Prothese in diese S-förmige Krümmung unter Umständen erschwert. Unter diesen Aspekten wird der Einbau des schaftes erleichtert, wenn das verjüngte Oberteil des Schaftblattes sich seinerseits in Richtung der Längsmittelachse nach proximal verjüngt; dem gleichen Zweck kann ein seitlich nach lateral ansteigender Uebergang der konischen Erweiterung zum verjüngten Oberteil dienen.

Die Klemmwirkung und die Abstützung des Schaftblattes lassen sich durch zusätzliche Massnahmen verbessern. So ist es vorteilhaft, die konische Erweiterung der Blattbreitseiten im unteren Schaftteil durch je eine wulstartige Mittelrippe zu bilden, die in parallel zu den Blattschmalseiten verlaufende Nuten übergeht; zweckmässigerweise kann man im Zusammenhang damit den rechteckigen Querschnitt des Schaftblattes in Form und Grösse so bemessen, dass sich der Schaft mit den vier abgerundeten Ecken des Rechteckes auf dem kortikalen Gewebe abstützt.

Rotationsbewegungen des Beines in der Hüfte werden hauptsächlich von der Gesässmuskulatur über den grossen Rollhöcker und den Schenkelhals auf den Hüftkopf und die Gelenkpfanne übertragen. Wird das Gelenk durch eine Prothese gebildet, so müssen die Drehmomente zunächst auf den Prothesenschaft übergeleitet werden, ehe eine Rotation des Prothesenkopfes in der künstlichen Gelenk-pfanne erfolgt. Auch diese Rotationsbeweg-ungen stellen hohe Anforderungen an die Stabilität des Implantates.

Die Rotationsstabilität kann entscheidend verbessert werden, wenn die laterale Blattschmalseite des verjüngten Oberteils des Schaftblattes mindestens annähernd vom Ende der konischen Erweiterung ausgehend zu einem Trochanterflügel ausgeweitet ist. Um dabei jedoch die Operationsöffnung im Trochanter möglichst klein zu halten und somit ein Maximum an präexistenter Spongiosa zu belassen, ist es weiterhin zweckmässig, den Trochanter-flügel nach lateral zu nochmals zu verjüngen.

Im folgenden wird die Erfindung anhand eines Ausführungsbeispiels im Zusammenhang mit der Zeichnung näher erläutert.

Fig. 1 ist eine Aufsicht auf die Blattseiten eines Schaftes für eine Hüftgelenkprothese;

Fig. 2 zeigt die Ansicht der Prothese nach Fig. 1 von lateral, während die

Fig. 3 bis 6 die Schnitte III—III bis VI—VI von Fig. 1 weidergeben.

Das Schaftblatt 1 des Prothesenschaftes erweitert sich von seinem distalen Ende 13 nach allen Seiten konisch, wobei der Konus symmetrisch zu einer Längsmittelachse 2 ausgebildet ist. Die mediale Schmalseite 3 des Konus geht in einen Bogen über, der an einem Kragen 4 endet.

Auf der anderen Seite des Kragens 4 ist über einen Hals 6 ein sich nach aussen konisch verjüngender Zapfen 7 aufgesetzt, der den nicht gezeigten kugelförmigen Gelenkkopf aufnimmt. Die Achse 8 des Zapfens 7 schneidet die Längsmittelachse 2 des Schaftes unter einem Winkel 9, der im wesentlichen den Winkel zwischen dem Schenkelhals und der Femurachse eines natürlichen Hüftgelenkes entspricht und im vorliegenden Fall 49° beträgt.

Auf den Blattseiten des Schaftblattes 1 wird die allseitige konische Erweiterung im wesentlichen durch—auf jeder Blattseite—je eine wulstartige Mittelrippe 14 gebildet, an die zum Rand der Blattseiten je eine Nut 15 anschliesst. Wie erwähnt, werden durch diese Ausgestaltung die Klemmwirkung und die Abstützung des Schaftes im Knochen gefördert. Längs- und Querabmessung des Schaftblattes 1 sowie seine Rechteckform mit abgerundeten Ecken 16 sind dabei so gewählt, das sich das Schaftblatt 1 auf der Schale des kortikalen Knochengewebes 17, wenn irgendwie möglich, mit allen vier Ecken 16 abstützt, wie dies in Fig. 6 rein schematisch angedeutet ist.

Die konische Erweiterung endet auf etwa 2/3 bis 3/4 der Schafthöhe und die wulstartigen Mittelrippen 14 gehen in das—mindestens relativ zu ihrer Dicke am oberen Ende—dünnere Oberteil 18 des Schaftblattes 1 über, wobei in dem genannten Höhenbereich des Schaftes auch die Nuten 15 auslaufen.

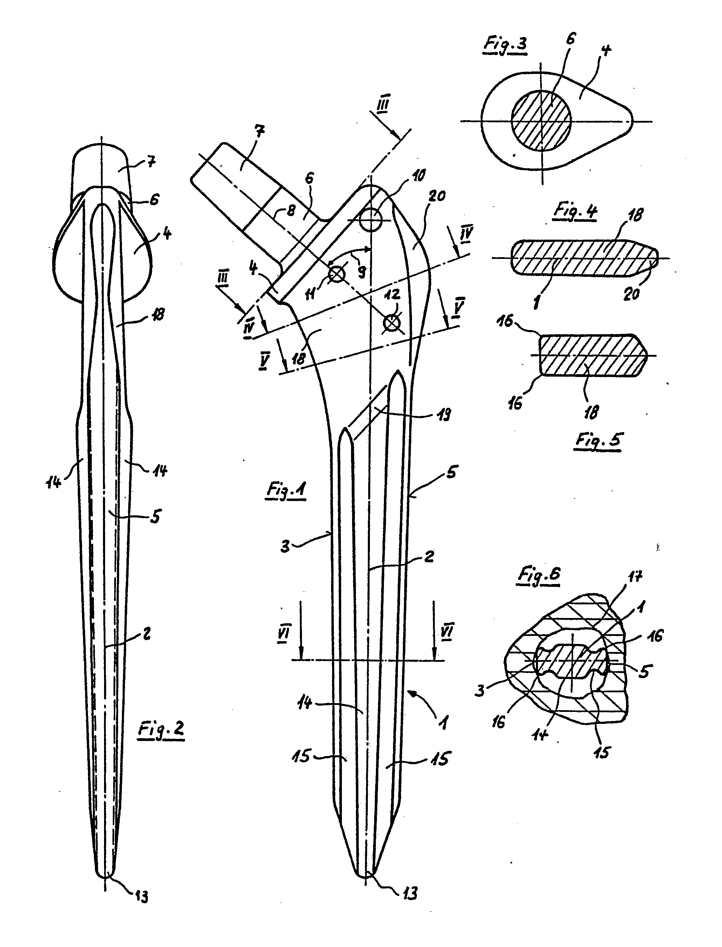

Das dünnere Oberteil 18 kann sich selbst zum Kragen 4 hin, wie in dem gezeigten Beispiel (Fig. 2), nochmals verjüngen oder über seine ganze Höhe die gleiche Dicke haben.

Wie Fig. 1 erkennen lässt, verläuft der Uebergang 19 von der konischen Erweiterung zum verjüngten Oberteil 18 auf der Blattseite nach lateral ansteigend, womit das Einsetzen der Prothese in der richtigen Stellung der Blattseiten erleichtert werden soll.

Die laterale Schmalseite 5 des Schaftblattes 1 geht—etwa auf der Höhe des Uebergangs 19—in einen Trochanter-Flügel 20 über, der sich—gegenüber dem verjüngten Oberteil 18—nochmals nach lateral verjüngt, im die für das Einsetzen des Flügels 20 notwendige Geweberesektion im Trochanter so gering wie möglich zu halten. Der Trochanterflügel 20, der von seiner äussersten lateralen Spitze in einem Bogen zum oberen Rand des Kragens 4 zurückgeführt ist, dient dazu, die Uebertragung von Drehmomenten von der Muskulatur über die Prothese auf das Hüftgelenk zu verbessern.

Durch das Schaftblatt 1 führen in seinem verjüngten Oberteil 18 drei Bohrungen 10—12, deren Mittelpunkte eine Dreiecksfläche einschliessen. Die Mittelpunkte der im Durchmesser gegenüber den beiden anderen 11 und 12 etwas grosseren Bohrung 10 liegt dabei auf der Längsmittelachse 2 des Schaftblattes 1, während die Bohrungen 11 und 12 auf der Zapfenachse 8 in einem genau vermessenen Abstand hintereinander angeordnet sind. Um Grösse und Lage der von den Bohrungen 10—12 eingeschlossenen Dreiecksfläche festzu-legen, ist darüberhinaus zusätzlich mindestens noch der senkrechte Abstand, den die Bohrung 10 von der Achse 8 hat, ausgemessen. Die Bohrungen 10—12 haben die Funktion, den Vergleich von zu verschiedenen Zeiten hergestellten Röntgenaufnahmen zu erleichtern. Ihre erwähnten Abstände können willkürlich gewählt werden; es ist jedoch zweckmässig, sie so gross wie—ohne Schwächung der mechanischen Eigenschaften des Schaftes, beispielsweise seiner Festigkeit—möglich zu wählen, da dadurch die relative Genauigkeit für ihre Messwerte auf den Bildern verbessert wird.

**Patentansprüche**

1. Blattartiger Schaft (1) für die Verankerung einer Hüftgelenkprothese im Femur, wobei der Schaft (1) sich vom distalen freien Ende (13) aus in Richtung seiner Längsmittelachse (2) bis in einen Bereich zwischen etwa 2/3 und 3/4 der Schafthöhe—gemessen entlang der Längsmittelachse (2)—allseitig konisch erweitert und wobei ferner seine mediale Schmalseite (3) aus dem Konus heraus in eine stetig gekrümmte Kurve übergeht, die an einem kragenartigen Ansatz (4) endet, der die Blattseiten vom Prothesenhals (6) trennt, dadurch gekennzeichnet, dass in Richtung quer zu den Blattbreitseiten das Oberteil (18) des Schaftblattes (1) bis zum kragenartigen Ansatz (4) gegenüber dem proximalen Ende (19) des Konus verjüngt ist.

2. Schaft nach Anspruch 1, dadurch gekennzeichnet, dass das verjüngte Oberteil (18) des Schaftblattes (1) sich seinerseits in Richtung der Längsmittelachse (2) nach proximal verjüngt.

3. Schaft nach Anspruch 1, dadurch gekennzeichnet, dass die laterale Blattschmalseite (5) im Bereich des Oberteils (18), mindestens annähernd vom Ende (19) der konischen Erweiterung ausgehend zu einem Trochanterflügel (20) ausgeweitet ist.

4. Schaft nach Anspruch 3, dadurch gekennzeichnet, dass der Trochanterflügel (20) vom Oberteil (18) aus sich nach lateral nochmals verjüngt.

5. Schaft nach Anspruch 1, dadurch gekennzeichnet, dass die konische Erweiterung der Blattbreitseiten durch je eine wulstartige Mittelrippe (14) gebildet ist, die in parallel zu den Blattschmalseiten (3, 5) verlaufende Nuten (15) übergeht.

6. Schaft nach Anspruch 1, dadurch gekennzeichnet, dass der Uebergang (19) von der konischen Erweiterung zum verjüngten Oberteil (18) des Schaftblattes (1) nach lateral ansteigt.

7. Schaft nach Anspruch 1, dadurch gekennzeichnet, dass der rechteckige Querschnitt des Schaftblattes (1) in Form und Grösse so bemessen ist, dass sich der Schaft mit den vier abgerundeten Ecken (16) des Rechteckes auf dem kortikalen Gewebe (17) abstützt.

**Revendications**

1. Tige du type lame (1) pour l'ancrage d'une prothèse d'articulation de la hanche dans le fémur, la tige (1) étant alors élargie de tous côtés coniquement depuis son extrémité libre distale (13) en direction de son axe longitudinal (2) jusque dans une zone se trouvant à peu près aux 2/3 et aux 3/4 de la hauteur de la tige, mesurée le long de l'axe longitudinal (2), et en outre son côté étroit médian (3) se transformant à partir du cône en une courbe de courbure constante qui se termine à un appendice (4) du type collet qui sépare les côtés de lame du col de la prothèse (6), caractérisée en ce que, dans la direction transversale par rapport aux côtés larges de lame, la partie supérieure (18) de la lame de tige (1) jusqu'à l'appendice du type collet (4) est rétrécie par rapport à l'extrémité proximale (19) du cône.

2. Tige selon la revendication 1, caractérisée en ce que la partie supérieure rétrécie (18) de la lame de tige (1) se rétrécit, pour sa part, du côté proximal en direction de l'axe longitudinal (2).

3. Tige selon la revendication 1, caractérisée en ce que le côté étroit de lame latéral (5) au voisinage de la partie supérieure (18) est élargi en une aile de trochanter (20) en partant au moins à peu près de l'extrémité (19) de l'élargissement conique.

4. Tige selon la revendication 3, caractérisée en ce que l'aile de trochanter (20) se rétrécit encore une fois latéralement à partir de la partie supérieure (18).

5. Tige selon la revendication 1, caractérisée en ce que l'élargissement conique des côtés larges de lame est formé par une nervure centrale respective (14) du type bourrelet, qui se transforme en rainures (15) s'étendant parallèlement aux côtés étroits de lame (3, 5).

6. Tige selon la revendication 1, caractérisée en ce que la transition (19), de l'élargissement conique à la partie supérieure (18) rétrécie de la lame de tige (1) monte latéralement.

7. Tige selon la revendication 1, caractérisée en ce que la section transversale rectangulaire de la lame de tige (1) est calculée en forme et en grandeur pour que la tige avec les quatre coins arrondis (16) du rectangle repose sur le tissu cortical (17).

**Claims**

1. A blade-like stem (1) for anchoring a hip joint prosthesis in the femur, the stem (1) widening conically in every direction from its distal free end (13) along its longitudinal central axis (2) as far as a zone disposed between approximately two-thirds and three-quarters of the way along stem length, as measured along said axis (2), the medial narrow side (3) of the stem emerging from the cone into a continuous curve terminating at a collar-like projection (4) which separates the blade sides from the prosthesis neck (6), characterised in that, in relation to the proximal end (19) of the cone, the top part (18) of the stem (1) narrows transversely of the wide sides of the stem as far as the collarlike projection (4).

2. A stem according to claim 1, characterised in that the narrow part (18) of the stem (1) in turn narrows proximally along the longitudinal centre axis (2).

3. A stem according to claim 1, characterised in that the lateral narrow side (5) of the stem widens near the top part (18) and starting at least substantially from the end (19) of the conical widening to form a trochanter blade (20).

4. A stem according to claim 3, characterised in that the trochanter blade (20) also narrows laterally from the top part (18).

5. A stem according to claim 1, characterised in that the conical widening of the wide sides of the stem takes the form in each case of a bead-like central rib (14) which merges into grooves (15) extending parallel to the narrow sides (3, 5) of the stem.

6. A stem according to claim 1, characterised in that the transition (19) between the conical widening and the narrowed top part (18) of the stem (1) rises laterally.

7. A stem according to claim 1, characterised in that the rectangular cross-section of the stem (1) is of a size and shape such that the stem bears by way of the four rounded corners (16) of the rectangle on the cortical tissue (17).

0 032 165

Fig. 3

Fig. 4

Fig. 5

Fig. 1

Fig. 2

Fig. 6

1